# EUROPEAN PATENT SPECIFICATION

(11) **EP 4 297 644 B1**
(45) Date of publication and mention of the grant of the patent: **15.01.2025**
(21) Application number: 22710817.2
(22) Date of filing: 23.02.2022
(51) Int. Cl.: A61B 5/06, A61B 5/283, A61M 25/09

(54) **ECG STYLET WITH IMPROVED FATIGUE AND BREAK RESISTANCE**
EKG-MANDRIN MIT VERBESSERTER ERMÜDUNGS- UND BRUCHFESTIGKEIT
STYLET D'ÉLECTROCARDIOGRAPHIE (ECG) PRÉSENTANT UNE RÉSISTANCE AMÉLIORÉE À LA FATIGUE ET À LA CASSE

(30) Priority: 23.02.2021 US 202163152746 P
(43) Date of publication of application: 03.01.2024
(73) Proprietor: Bard Access Systems, Inc., Salt Lake City, UT 84116 (US)
(72) Inventor: AKINS, Samuel, Draper, Utah 84020 (US); DAVIS, Michael, West Jordan, Utah 84081 (US); LACKEY, Breanna E., West Valley City, Utah 84119 (US); BURKHOLZ, Jonathan Karl, Salt Lake City, Utah 84108 (US)
(74) Representative: Hoffmann Eitle
(86) International application number: PCT/US2022/017568
(87) International publication number: WO 2022/182790

(56) References cited:
- US-A1- 2018 169 389
- US-A1- 2018 304 043

## Description

### PRIORITY

This application claims the benefit of priority to U.S. Provisional Application No. 63/152,746, filed February 23, 2021.

### BACKGROUND

Various endovascular devices, including without limit central venous catheters ("CVC"), may be inserted into the vasculature of a patient to detect and/or treat various health issues. CVCs are endovascular devices including any catheter designed to utilize the central veins (e.g., subclavian and superior vena cava) or right sided cardiac chambers for the delivery and/or withdrawal of blood, blood products, therapeutic agents, and/or diagnostic agents. CVCs also include catheters inserted into the central veins or right sided cardiac chambers for the acquisition of hemodynamic data. Standard central venous catheters for intravenous access, dialysis catheters, percutaneously introduced central catheters ("PICC" lines), and right heart catheters are examples of CVCs. In some applications, an endovascular device, e.g., a central venous catheter (CVC), may be inserted into the superior vena cava (SVC) of a patient.

The specific location placement of an endovascular device is very important and can have a significant impact on the health of the patient. For example, a central venous catheter (CVC) with its tip located in the ideal position provides reliable vascular access with optimal therapeutic delivery, while minimizing short and long-term complications.

While CVCs have been used for many years, determining the position of the tip of the CVC has always been problematic. Further, in addition to the need to know where the tip is during initial placement, the CVC may migrate or otherwise move after the initial placement and require re-positioning. Therefore, the operator must monitor or periodically reevaluate the location of the tip.

Electrocardiogram (ECG) based guidance can be used as a positioning technique for catheter tip placement and confirmation. The electrical conduction system of the heart creates specific electrical signals, electrical energy distributions and behaviors thereof which are indicative of specific locations in the thoracic cavity and/or of specific heart functions or conditions. When measured endovascularly or intravascularly, i.e., from within blood vessels or from within the heart, certain parameters of the electrical activity of the heart can be used to identify specific locations in the cardiovascular system and/or functional conditions, normal or abnormal.

Some catheter guidance systems may also include a tip location/navigation system ("TLS") modality for magnetically-based tracking of the catheter tip. Such system may include magnetic elements coupled to a stylet within the catheter to be placed within the vasculature of the patient. In some instances, the magnetic elements may potentially become decoupled from the stylet exposing the patient to particulate emboli.

US 2018/0169389 A1 relates to a stylet for use in guiding a distal tip of a catheter to a predetermined location within the body of a patient. In one embodiment the stylet is configured for use within a lumen of the catheter and comprises a core wire, an ECG sensor, and a magnetic assembly. In one embodiment a distal segment of the stylet includes a tubing inside which is disposed a distal portion of the core wire, terminating at the core wire distal end. A conductive wire proximally extends within the tubing from the stylet distal end to the proximal end of the stylet for connection with a suitable ECG sensor module. The magnetic assembly, including a plurality of permanent magnets or other suitable magnetic/electromagnetic elements, is disposed distally to the core wire. A conductive coil proximally extends within the tubing and about the magnetic assembly from the stylet distal end to a connection point with the core wire at the proximal end of the coil.

Disclosed herein are new devices and methods for enhancing the reliability of stylets for use with magnetically-based tracking systems thereby reducing the probability of magnetic elements becoming decoupled from the stylet and providing enhanced safety for the patient against exposure to particulate emboli.

### SUMMARY

Disclosed herein is a stylet for placing a catheter in a vasculature of a patient. The stylet includes an ECG sensor assembly having an electrode extending from a proximal end to a distal end of the stylet and the proximal end is configured to couple with an ECG sensor. The stylet further includes a magnetic assembly disposed along a distal portion of the stylet and the magnetic assembly producing a magnetic field. The stylet further includes a core wire extending proximally away from the magnetic assembly and a coil defining a lumen. The coil extends along a distal portion of the stylet, and a distal portion of the core wire is disposed within the lumen of the coil.

The stylet may be configured to be inserted within a lumen of the catheter and the stylet may also be configured for placement of the catheter within a superior vena cava of the patient. The electrode may include the core wire and the coil.

The magnetic assembly includes a plurality of magnet elements disposed within the lumen of the coil. Each magnet element may have a cylindrical shape, and the magnet elements may be arranged end to end within the lumen. One or more of the magnet elements are attached to the coil.

The coil may be attached to the core wire and may also be electrically coupled with the core wire. The coil may include a coil member forming a helix. The coil member may have a rectangular cross-sectional shape with a width and a thickness.

The coil may include a second coil member forming at least a second helix, and the first coil member and the second coil member may cross each other. In some embodiments, the coil includes at least three coil members defining a braided or woven structure. The stylet may include a sheath extending along the distal portion of the stylet and the sheath may cover the coil.

The core wire may have a first thickness extending along a proximal portion of the core wire and a second thickness extending along a distal portion of the core wire and the second thickness may be less than the first thickness. The core wire may include a taper extending between the first thickness the second thickness. The distal portion of the core wire may be round, and in some embodiments, the distal portion of the core wire extends along the magnet assembly.

The stylet may include a distal tip member coupled with the coil and the distal tip member may be formed of an electrically conductive material. The distal tip member may also be electrically coupled with the coil.

The distal portion of the core wire may extend distally beyond the magnet assembly, and the core wire may be electrically coupled with the core wire.

The stylet may further include a handle attached to the core wire at a proximal end of the core wire and the stylet may also further include a tether coupled with the core wire at the proximal end of the core wire, where the tether includes an electrical conductor forming a portion of the electrode.

The stylet may have a width is between 0.01 mm and 1 mm and a thickness between 0.005 mm and 1 mm. A pitch of the helix may be between 0.01 mm and 10 mm.

Also disclosed herein is an intravascular catheter assembly. The catheter assembly includes a catheter having a lumen and a stylet disposed within the lumen of the catheter. The stylet includes an ECG sensor assembly having an electrode extending from a proximal end to a distal end of the stylet and the proximal end is configured to couple with an ECG sensor. The stylet further includes a magnetic assembly disposed along a distal portion of the stylet and the magnetic assembly producing a magnetic field. The stylet further includes a core wire extending proximally away from the magnetic assembly and a coil defining a lumen. The coil extends along a distal portion of the stylet, and a distal portion of the core wire is disposed within the lumen of the coil.

The catheter assembly may be configured for placement of a tip of the catheter within superior vena cava of a patient and a distal end of the catheter may be substantially co-terminal with a distal end of the stylet.

The catheter assembly may include a preformed curve along a distal portion of the catheter assembly, and the preformed curve may be defined by a preform curve of the stylet.

The coil of the stylet may be entirely disposed within the lumen and a sheath of the stylet may also be entirely disposed within the lumen.

Also disclosed herein is method a placing a catheter within a superior vena cava of a patient. The method includes inserting a stylet within a lumen of the catheter. The stylet includes an ECG sensor assembly including an electrode extending from a proximal end to a distal end of the stylet, where the electrode is configured to transmit ECG signals to an ECG system. The stylet further includes a magnetic assembly producing a magnetic field and an elongate coil extending proximally away from the distal end of the stylet. The coil defines a lumen that contains the magnetic assembly.

The method further includes connecting the ECG sensor assembly to an ECG system, advancing the catheter along a vasculature of the patient, discontinuing advancement of the catheter upon an indication via an ECG signal that a tip of the stylet is disposed within the superior vena cava, and removing the stylet from the lumen of the catheter.

In some embodiments of the method, the stylet includes a handle and the method further includes manually applying a torque to the handle to rotate the stylet within the catheter. The method may further include manually applying a torque to the handle and the catheter to rotate the catheter within the vasculature.

The method may further include aligning a distal tip of the stylet with a distal tip of the catheter, and transmitting an ECG signal along a conductive coil member of the coil.

These and other features of the concepts provided herein will become more apparent to those of skill in the art in view of the accompanying drawings and following description, which describe particular embodiments of such concepts in greater detail.

### BRIEF DESCRIPTION OF THE DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 is a simplified view of a patient and a catheter being inserted therein with assistance of an integrated system, in accordance with some embodiments.
FIG. 2 is a perspective view of a stylet employed in connection with the integrated system of FIG. 1, in accordance with some embodiments.
FIG. 3A is a side view of a catheter engagement section of the stylet of FIG. 2, in accordance with some embodiments.
FIG. 3B is a detail view side view of a portion of the catheter engagement section at a junction point, in accordance with some embodiments.
FIG. 3C is a cross-sectional side view of a transition portion of the catheter engagement section, in accordance with some embodiments.
FIG. 3D is a cross-sectional side view of a distal tip portion of the catheter engagement section, in accordance with some embodiments.
FIG. 4 illustrates a catheter assembly including the catheter and the stylet of FIG. 1, in accordance with some embodiments.

### DETAILED DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

The directional terms "proximal" and "distal" are used herein to refer to opposite locations on a medical device. The proximal end of the device is defined as the end of the device closest to the end-user and further from the patient when the device is in use by the end-user. The distal end is the end opposite the proximal end, along the longitudinal direction of the device, or the end furthest from the end-user and more near the patient.

Unless defined otherwise, all technical and scientific terms used herein have the same meaning as commonly understood by those of ordinary skill in the art.

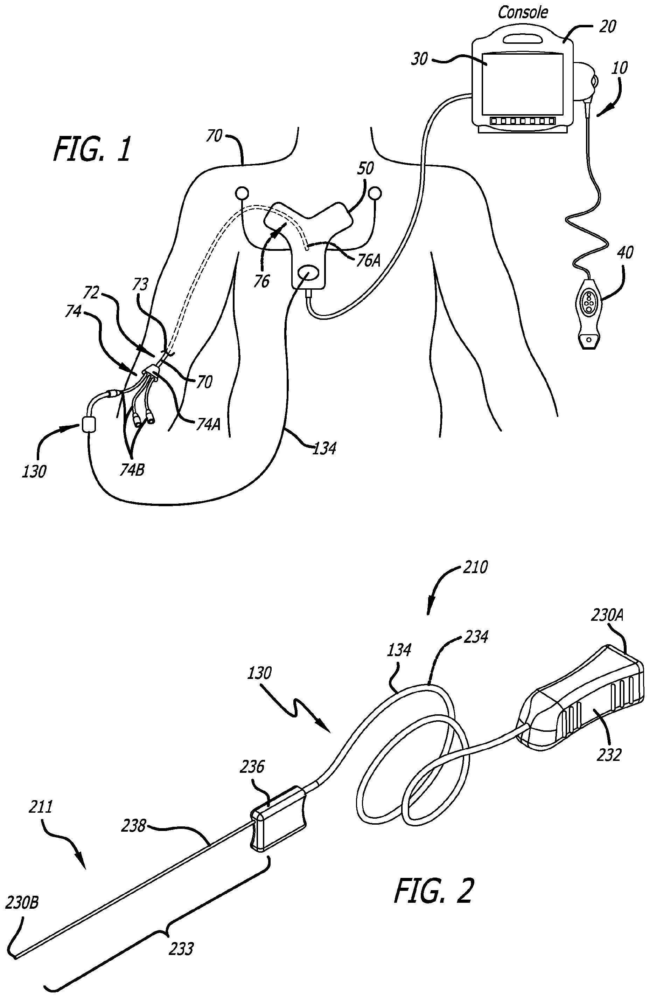

FIG. 1 depicts various features of a catheter placement system ("system") 10, which is generally directed to a catheter placement system configured for accurately placing a catheter within the vasculature of a patient 70. The catheter placement system 10 employs three modalities for improving catheter placement accuracy: 1) ultrasound-assisted guidance for introducing the catheter into the patient's vasculature; 2) a tip location/navigation system ("TLS") for magnetically-based tracking of the catheter tip; and 3) ECG signal-based catheter tip guidance. The combination of the three modalities above enables the catheter placement system 10 to facilitate catheter placement within the patient's vasculature with a relatively high level of accuracy, i.e., placement of the distal tip of the catheter in a predetermined and desired position. Moreover, because of the ECG-based guidance of the catheter tip, correct tip placement may be confirmed without the need for a confirmatory X-ray. This, in turn, reduces the patient's exposure to potentially harmful x-rays, the cost and time involved in transporting the patient 70 to and from the x-ray department, costly and inconvenient catheter repositioning procedures, etc.

The combined features of the system 10 are integrated into a single device for use by a clinician placing the catheter 72. Integration of the three modalities into a single device simplifies the catheter placement process and results in relatively faster catheter placements. The integrated catheter placement system 10 enables ultrasound, TLS, and ECG activities to be viewed from a single display of the integrated system. Some systems and methods of TLS and ECG based guidance are described in US 9,220,432, titled "Method and system of utilizing ECG signal for central venous catheter tip positioning," and US 9,999,371, titled "Integrated system for intravascular placement of a catheter." Additional disclosure of stylets and catheters for use with a TLS system and ECG based guidance can be found in the following U.S. patents: US 8,388,541; US 8,781,555; US 8,784,336; US 8,849,382; US 9,636,031; US 9,649,048; and US 9,901,714.

FIG. 1 further depicts various components of the system 10, including a console 20, display 30, probe 40, and sensor 50. FIG. 1 shows the general relation of these components to a patient 70 during a procedure to place a catheter 72 into the patient vasculature through a skin insertion site 73. The catheter 72 generally includes a proximal portion 74 that remains exterior to the patient 70 and a distal portion 76 that resides within the patient vasculature after placement is complete. The system 10 is employed to ultimately position a distal tip 76A of the catheter 72 in a desired position within the patient vasculature. In some embodiments, the desired position for the catheter distal tip 76A is proximate the patient's heart, such as in the lower one-third (1/3rd) portion of the Superior Vena Cava ("SVC"). Of course, the system 10 can be employed to place the catheter distal tip 76A in other locations. The catheter proximal portion 74 further includes a hub 74A that provides fluid communication between the one or more lumens of the catheter 72 and one or more extension legs 74B extending proximally from the hub.

A stylet 130 is removably loaded into the catheter 72 and employed during insertion to position the distal tip 76A of the catheter in a desired location within the patient vasculature. The stylet 130 may be pre-loaded within a lumen of the catheter 72 in one embodiment such that the distal end 130B is substantially flush, or co-terminal, with the catheter opening at the distal end 76A thereof. Note that, though described herein as a stylet, in other embodiments a guidewire or other catheter guiding apparatus could include the principles of the embodiment described herein.

FIG. 2 shows the stylet 130 removed from the catheter 72. Reference is now made to FIG. 2 in describing various details of the stylet 130. As shown, the stylet 130 defines a proximal end 230A and a distal end 230B and includes an ECG sensor assembly 210 and a magnetic assembly 211. A connector 232 is included at the proximal end 230A, and the tether 134 extends distally from the connector 232 and attaches to a handle 236. A core wire 238 extends distally away from the handle 236. Each of the assemblies and components of the stylet 130 are described in detail below.

The handle 236 is provided to enable insertion/removal of the stylet 130 from the catheter 72. In embodiments where the core wire 238 is torqueable, the handle 236 further enables the core wire 238 to be rotated within the lumen of the catheter 72, to assist in navigating the catheter distal portion through the vasculature of the patient 70.

The handle 236 attaches to a distal end of the tether 134. In the present embodiment, the tether 134 is a flexible, shielded cable housing one or more conductors 234 (e.g., wires) electrically connected to both the core wire 238 and the connector 232. As such, the tether 134 provides a conductive pathway from the distal portion of the core wire 238 through to the tether connector 232 at proximal end 230A of the stylet 130. The connector 232 may be configured for operable connection to the TLS sensor 50 on the patient's chest for assisting in navigation of the catheter distal tip 76A to a desired location within the patient vasculature. A catheter engagement section 233 of the stylet 130 extends between the distal end 230B and the handle 236.

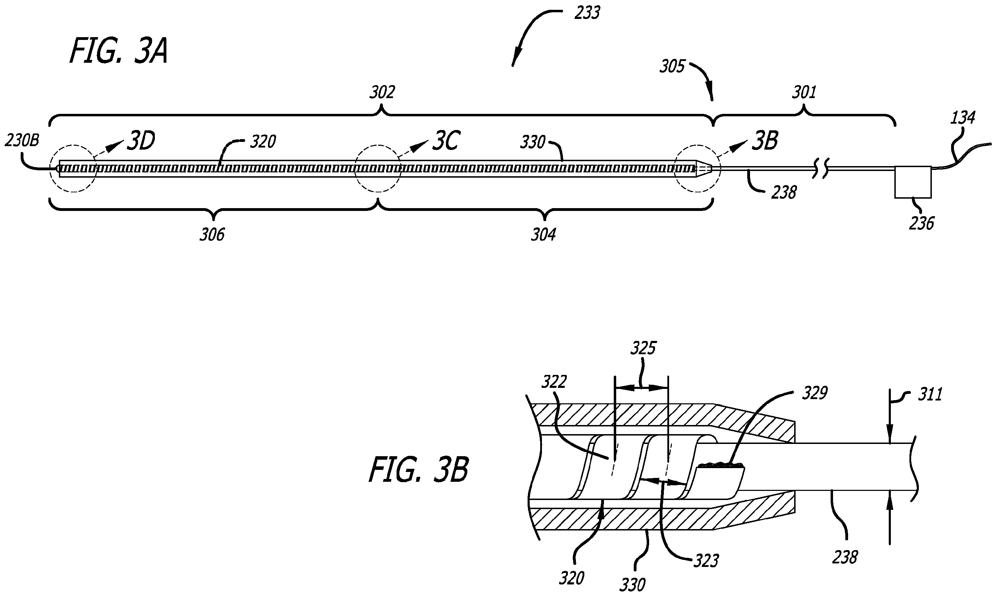

FIGS. 3A-3D illustrate details of the catheter engagement section 233 of stylet 130. FIG. 3A illustrates a side view of the catheter engagement section 233. As shown in FIG. 3A, the catheter engagement section 233 includes a proximal portion 301 and a distal portion 302. The proximal portion 301 extends distally from the handle 236 to a junction point 305 and the distal portion 302 extends distally from the junction point 305 to the distal end 230B. The distal portion 302 includes a distal tip portion 306 extending proximally away from the distal end 230B and a transition portion 304 extending distally away from the junction point 305.

As described above, the stylet 130 includes a core wire 238 defining an elongate shape extending distally away from the handle 236 along the proximal portion 301 and at least partially along the distal portion 302. A coil 320 and a sheath 330 extend along the distal portion 302 as further described in detail below. The core wire 238 is composed of a suitable stylet material including stainless steel or a memory material such as, in one embodiment, a nickel and titanium-containing alloy commonly known by the acronym "nitinol." Although not shown here, in some embodiments, the stylet 130 may include one or more pre-shaped (e.g., curved) configurations along the catheter engagement section 233 so as to urge the distal portion of the catheter 72 into similar corresponding pre-shaped configurations. In other embodiments, the core wire 238 includes no pre-shaping.

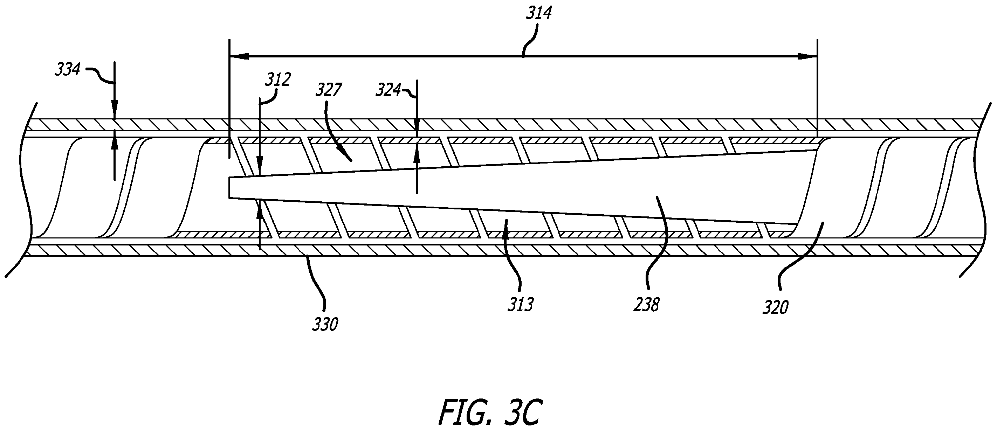

FIG. 3B is a detail view of the catheter engagement section 233 at the junction point 305. As shown in FIG. 3B, the core wire 238 extends proximally away from the junction point 305 having a first cross-sectional diameter 311. The coil 320 and the sheath 330 extend distally away from the junction point 305. A distal portion of the core wire 238 extends distally away from the junction point 305 and is disposed within a lumen 327 of the coil 320 (as shown in FIG. 3C). In some embodiments, the sheath 330 extend proximally away from the junction point 305 covering at least a portion of the core wire 238.

The coil 320 extends along the distal portion 302 from the junction point 305 to the distal end 230B. The coil 320 defines flexibility and stiffness characteristics of the distal portion 302 so that the stylet 130 may follow a curved pathway of the vasculature without causing injury to the internal wall of the vasculature. The coil 320 may also define robustness and/or fatigue resistance characteristics of the distal portion 302. In other words, the coil 320 may define a reliability of the stylet 130 against breakage during use. More specially, the coil 320 may prevent breakage of the distal portion 302 should the distal portion 302 be bent one or more times during use.

Referring to FIGS. 3B and 3C, the coil 320 is formed of a coil member 322. In some embodiments, the coil 320 may include two or more coil members 322. The structural properties (i.e., the cross-sectional shape and material) of the coil member 322 may at least partially define the flexibility and/or robustness of the distal portion 302. For example, a coil member 322 having a thick cross-section may define a flexibility and/or robustness of the distal portion 302 that is less than a coil member 302 having a thin (i.e., less thick) cross-section. A cross-sectional shape of the coil member 322 may be round or non-round. In the illustrated embodiment, the coil member includes a rectangular cross-sectional shape having a width 323 and a thickness 324. In the illustrated embodiment, the width 323 of the coil member 322 may be between about 0.01 mm and 0.5 mm. In other embodiments, the width 323 may be between about 0.01 mm and 0.3 mm, 0.05 mm and 0.3 mm, 0.1 mm and 0.3 mm, or 0.01 mm and 1 mm. In the illustrated embodiment, the thickness 324 of the coil member 322 may be between about 0.005 mm and 0.3 mm. In other embodiments, the thickness 324 may be between about 0.01 mm and 0.3 mm, 0.05 mm and 0.3 mm, 0.05 mm and 0.2 mm or 0.01 mm and 1 mm. In some embodiments, the coil member 322 may include a round shape. In such embodiments, the diameter may be between about 0.005 mm and 0.3 mm. In other embodiments, the diameter may be between about 0.01 mm and 0.3 mm, 0.05 mm and 0.3 mm, or 0.05 mm and 0.2 mm. In some embodiments, the coil member 322 may be formed of multiple wire filaments forming a cable, such as a braided cable, for example.

The coil 320 also defines a pitch 325, i.e., spacing of adjacent windings of the coil 320. In some embodiments, the coil 320 may be configured so that windings of the coil 320 are located immediately adjacent one another, i.e., so that adjacent windings are in contact with each other. In other embodiments, the coil 320 may be configured to define a space or separation between adj acent windings. In some embodiments, the pitch 325 of the coil 320 may be between about 0.01 mm and 10 mm. In other embodiments, the pitch 325 may be between about 0.01 mm and 1 mm, 0.01 mm and 0.5 mm, or 0.1 mm and 0.3 mm.

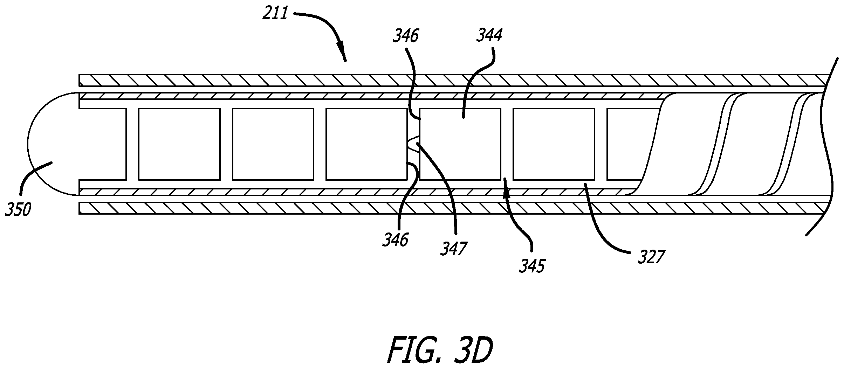

In some embodiments, the width 323 and/or pitch 325 of the coil 320 may vary along the distal portion 302, i.e., the length of the coil 320. In some embodiments, the width 323 and/or pitch 325 may be inter-related. For example, a longer width 323 may define a longer pitch 325 and a shorter width 323 may define a shorter pitch 325. As may be appreciated by one of ordinary skill, the width 323 combined with the pitch 325 may at least partially, and in some embodiments substantially define, a flexibility and/or robustness of the stylet 130 along the distal portion 302. As the distal portion 302 is advanced along the vasculature, the distal portion 302 may assume a curved shape having different bending radii. For example, the bending radius along the distal tip portion 306 may be shorter than a bending radius along the transition portion 304. As such, it may be advantageous for the coil 320 to have a shorter width 323 and a corresponding shorter pitch 325 along the distal tip portion 306 than along the transition portion 304 to define a greater flexibility and/or robustness along the distal tip portion 306. By varying the width 323 and/or pitch 325 along the distal portion 302, the stiffness, flexibility, and robustness may be optimized along the distal portion 302. In a similar fashion, varying the width 323 of the coil member 322 may also define stiffness, flexibility, and robustness. Further description regarding varying pitch and varying width is provided below in relation to FIG. 3D.

In the illustrated embodiment, coil 320 includes a single coil member 322 forming a single helix. In other embodiments, the coil 320 may include two, three, four, or more coil members 322 arranged in helical orientations. In the illustrated embodiment, the single coil member 322 defines a single coil layer. In other embodiments, two or more coil members 322 may be co-wound with respect to each other defining a single coil layer. In other embodiments, two or more coil members 322 may be co-wound or counter-wound with respect to each other defining more than one layer. In some embodiments, three or more coil members 322 may be arranged to define a braided or woven structure of the coil 320.

The coil member 322 may be formed of a metallic material such as stainless steel or nitinol (see above). In some embodiments, one or more coil members 322 may be formed for a polymeric material. In the illustrated embodiment, the coil 320 may be configured to conduct electricity from a proximal end to a distal end of the coil 320.

The coil 320 is physically coupled with the core wire 238. The coupling between the coil 320 and the core wire 238 may define an electrical connection between the coil 320 and the core wire 238. In the illustrated embodiment, the coil 320 may be attached to the core wire 238 via a weld 329 at a proximal end of the coil 320. In some embodiments, the coupling may include a radial clamping force of the coil 320 on the core wire 238 defined by interfering dimensions. In other words, in a free state, an inside diameter of the coil 320 may be less than the first diameter 311 of the core wire 238 so that the coil 320 exerts a radial inward clamping force on the core wire 238 when assembled, i.e. when the core wire 238 is disposed within the lumen 327 of the coil 320. In other embodiments, the coil 320 may be attached to the core wire 238 via an adhesive. As may be appreciated by one of ordinary skill, the coil 320 may be attached to the core wire 238 via any other suitable attachment method and at one or more other locations along an over lapping length of the core wire 238 and the coil 320.

FIG. 3C illustrates a side view of the transition portion 304 of the distal portion 302 with portions of the coil 320 and sheath 330 shown in cross-section. As stated above, a distal portion of the core wire 238 is disposed within the lumen 327 of the coil 320. Along the distal portion, the core wire 238 may distally transition from the first diameter 311 to a second diameter 312. In the illustrated embodiment, the transition may be defined by a taper 313. In some embodiments, the flexibility and or stiffness of the distal portion 302 may be defined by a combination of a flexural stiffness of the core wire 238 and a flexural stiffness of the coil 320 along the transition portion 304. As may be appreciated by one of ordinary skill, the stiffness of the core wire 238 decreases with a decrease in diameter of the core wire 238. In other words, the stiffness of the catheter engagement section 233 may gradually decrease along the transition portion 304. The first diameter 311 of the core wire 238 may be between about 0.01 mm and 0.5 mm. In other embodiments, the first diameter 311 may be between about 0.01 mm and 0.3 mm, 0.05 mm and 0.3 mm, or 0.1 mm and 0.3 mm. Similarly, the second diameter 312 may be between about 0.01 mm and 0.5 mm. In other embodiments, the second diameter 312 may be between about 0.01 mm and 0.3 mm, 0.05 mm and 0.3 mm, or 0.1 mm and 0.3 mm. A longitudinal taper length 314 of the taper 313 may be between about 3 mm and 30 mm. In other embodiments, the taper length 314 may be between about 10 mm and 30 mm, 20 mm and 30 mm, or 23 mm and 27 mm.

The sheath 330 is disposed along an exterior of the coil 320 from the junction point 305 to the distal end 230B. The sheath 330 may provide for a smooth outside surface and/or a low-friction outside surface of the distal portion 302 to facilitate insertion of the distal portion 302 within the catheter 72. The sheath 330 may be formed of an extruded tube into which the coil 320 is inserted during assembly. In some embodiments, sheath material may be applied to the coil 320 in a liquid state so that the sheath 330 is formed upon curing/hardening of the sheath material. In other embodiments, the sheath 330 may be formed of a shrinkable tube. In such an embodiment, the assembly process may include placing the coil 320 within the shrinkable tube and thereafter shrinking the shrinkable the tube onto the coil 320. In still other embodiments, the sheath 330 may be formed of a tape wrapped around the coil 320. The sheath material may include polyethylene, polypropylene, polytetrafluoroethylene, polyimide or any other suitable polymeric material. In some embodiments, the sheath 330 may contribute to the stiffness of the distal portion 302. For example, the sheath 330 may facilitate defining a preform shape of the distal portion 302.

While the illustration of FIG. 3B shows an outside diameter of the sheath being greater than the first diameter 311 proximal of the sheath 330, in some embodiments, the catheter engagement section 233 may include a substantially constant cross-sectional size (e.g., diameter) across the junction point 305. An outside diameter of the sheath 330 may be substantially equal to the first diameter 311 of the core wire 238. In such instances, the first diameter 311, the taper 313, the coil member thickness 324, and a sheath thickness 334 may be sized and/or longitudinally positioned to define a substantially constant outside diameter across the junction point 305.

FIG. 3D is a cross-sectional detail illustration of the distal tip portion 306. As stated above, the stylet 130 includes the magnetic assembly 211 disposed along distal tip portion 306 proximate the distal end 230B. The magnetic assembly 211 may be configured for use during TLS mode of the system 10. The magnetic assembly 211 includes a plurality of magnetic elements 344 disposed within the lumen 327 of the coil 320. The plurality of magnetic elements 344 may form a linear array of magnetic elements 344 extending proximally away from the distal end 230B. In the illustrated embodiment, the magnetic elements 344 include 20 ferromagnetic magnets of a solid cylindrical shape stacked end-to-end such that end faces 346 of the magnetic elements 144 are disposed adjacent one another. In other embodiments, however, the magnetic element 344 may vary from this design in not only shape, but also composition, number, size, magnetic type, and position within the lumen 327 and along the distal tip portion 306. One or more magnetic elements 344 are attached to the coil 320 so that longitudinal displacement of the magnetic elements 344 with the lumen 327 may be constrained.

In some embodiments, the magnetic assembly 211 may include a space or separation 345 between adjacent end faces 346 of the magnetic elements 344. The space 345 may facilitate a reduced stress and/or strain of the coil 320 and/or the sheath 330 when the distal tip portion 306 is disposed in a curved shape. A reduction in stress or strain along the distal tip portion 306 may inhibit breakage of the stylet 130 along the distal tip portion 306 thereby enhancing reliability of the stylet 130. In some embodiments, the space 345 may be defined by a centralized extension 347 extending away from one or both end faces 346 of one or more magnetic elements 344. In some embodiments, the centralized extension 347 may take the form of a radius or chamfer on one or both end faces 346.

The magnetic elements 144 are employed along the distal tip portion 306 to enable the position of the stylet distal end 230B to be observable relative to the TLS sensor 50 placed on the patient's chest (see FIG. 1). The TLS sensor 50 is configured to detect the magnetic field of the magnetic elements 344 as the stylet 130 advances with the catheter 72 through the patient vasculature. In this way, a clinician placing the catheter 72 is able to generally determine the location of the catheter distal end 76A within the patient vasculature and detect when catheter malposition is occurring, such as advancement of the catheter along an undesired vein, for instance.

As illustrated in FIG. 3D, the stylet 130 may include a distal tip member 350 disposed at the distal end 230B. The distal tip member 350 may extend beyond distal ends of one or both of the coil 320 and the sheath 330. The distal tip member 350 may be attached to the coil 320. The distal tip member 350 may also be electrically coupled with the coil 320. The distal tip member 350 is formed of an electrically conductive material. In some embodiments, the distal tip member 350 may be formed of a metallic material such as stainless steel or any other suitable metallic material. In such embodiments, the distal tip member 350 may be welded to the coil 320. In other embodiments, the distal tip member 350 may be formed of a non-metallic material having electrical conduction properties. For example, the distal tip member 350 may include a conductive epoxy. The distal tip member 350 may at least partially define and/or increase a conductive surface of the distal end 230B of the stylet 130 so as to improve the ability of the stylet 130 to detect ECG signals.

The connector 232, the conductors 234, the core wire 238, the coil 320, and the distal tip member 350 are all in electric communication with each other to define the ECG sensor assembly 210 including an electrical conduction pathway for transmission of ECG signals from the distal end 230B to the proximal end 230A of the stylet 130. As such, ECG sensor assembly 210 defines an electrode to facilitate transmission of EGC signals from a fluid of the patient 70 (e.g., blood within the superior vena cava) to the ECG sensor 50. The ECG sensor assembly 210 enables the stylet 130, disposed in a lumen of the catheter 72 during insertion, to be employed in detecting an intra-atrial ECG signal produced by an SA or other node of the patient's heart, thereby allowing for navigation of the distal tip 76A of the catheter 72 to a predetermined location within the vasculature proximate the patient's heart. Thus, the ECG sensor assembly 210 serves as an aide in confirming proper placement of the catheter distal tip 76A.

In some embodiments, the core wire 238 may extend distally to the distal end 230B. In such embodiments, a portion of the core wire 238 may be disposed between the magnetic elements 344 and an inside luminal surface of the coil 320. The core wire 238 is electrically coupled with the distal tip member 350 directly and as such, the core wire 238 need not be electrically coupled with the coil 320.

As discussed above in relation to FIG. 3B, the pitch of the coil 320 and/or the width of the coil member 322 may vary along the distal portion 302. As such, in some embodiments, the pitch may vary along the distal portion 302 between the pitch 325 adjacent the junction point 305 as shown in FIG. 3B and a pitch 335 adjacent the distal end 230B as shown FIG. 3D. In some embodiments, the pitch 335 may be less than the pitch 325 to define a flexibility and/or a robustness of the stylet 130 at the distal end 230B that is greater than the flexibility and/or robustness adjacent the junction point 305. In some embodiments, the pitch 335 may extend along the distal tip portion 306 and the pitch 325 may gradually transition toward the pitch 335 along the transition portion 304.

Similarly, in some embodiments, the width of the coil member 322 may vary along the length of the distal portion 302 between the width 323 adjacent the junction point 305 as shown in FIG. 3B and a width 333 adjacent the distal end 230B as shown FIG. 3D. In some embodiments, the width 333 may be less than the width 323 to define a flexibility and/or a robustness of the stylet 130 at the distal end 230B that is greater than the flexibility and/or the robustness adjacent the junction point 305. In some embodiments, the width 333 may extend along the distal tip portion 306 and the width 323 may gradually transition toward the width 333 along the transition portion 304.

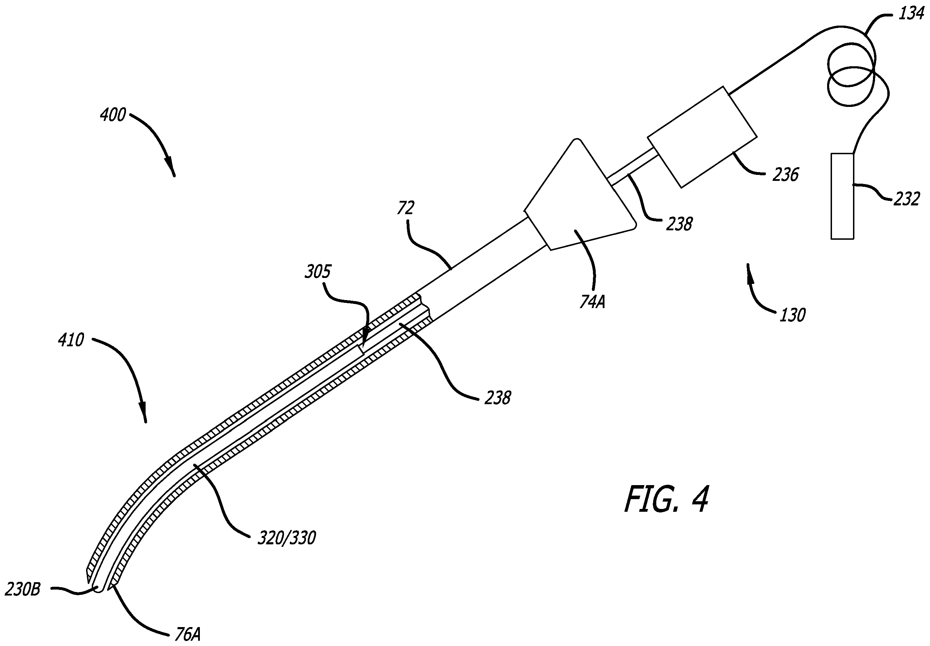

FIG. 4 illustrates a catheter assembly 400 including the catheter 72 having the stylet 130 disposed within a lumen of the catheter 72. In some embodiments, the stylet 130 may be disposed with the catheter 72 during manufacturing. In other embodiments, the clinical may insert the stylet 130 within the catheter 72 prior to inserting the catheter into the patient vasculature. In the illustrated embodiment, the stylet 130 is disposed within the catheter 72 such that the distal end 230B of the stylet 130 is substantially co-terminal with the distal tip 76A of the catheter 72, thus placing the distal tips of both the stylet and the catheter in substantial alignment with one another. In other embodiments, the distal end 230B of the stylet 130 may not be substantially co-terminal with the distal tip 76A of the catheter 72.

In the illustrated embodiment, the catheter assembly 400 includes a preformed curve 410. The preformed curve 410 may be defined by a preformed curve of the stylet 130, a preformed curve of the catheter 72, or both. In some embodiments of use, the clinician may apply a torque to the handle 236 to rotate the stylet 130 with respect to the catheter 72. Doing so, may orient the curve 410 in a different direction with respect to the hub 74A of the catheter 72. In other embodiments of use, the clinician may simultaneously apply a torque to the handle 236 and to the catheter 72 to rotate the catheter assembly 400 within the vasculature of the patient 70, thereby reorienting the curve 410 with respect to the patient 70. In some embodiments, the catheter assembly 400 may include more than one preformed curve 410. In other embodiments, the preformed curve 410 may be omitted.

In the illustrated embodiment, the stylet 130 is inserted within the catheter 72 sufficiently to dispose the junction point 305 within the catheter 72. In this embodiment, the coil 320 and the sheath 330 are entirely disposed within the catheter 72. In other embodiments, the junction point 305 may be disposed external to the catheter 72, i.e., proximal the catheter hub 74A, so that only a portion of the coil 320 and the sheath 330 are disposed within the catheter 72.

Any methods disclosed herein include one or more steps or actions for performing the described method. The method steps and/or actions may be interchanged with one another. In other words, unless a specific order of steps or actions is required for proper operation of the embodiment, the order and/or use of specific steps and/or actions may be modified.

In use, the stylet 130 may be loaded into a lumen of the catheter 72 to define a catheter assembly before catheter placement. Note that the stylet 130 can come preloaded in the catheter lumen from the manufacturer, or loaded into the catheter by the clinician prior to catheter insertion. The stylet 130 is disposed within the catheter lumen such that the distal end 230B of the stylet 130 is substantially co-terminal with the distal tip 76A of the catheter 72, thus placing the distal tips of both the stylet and the catheter in substantial alignment with one another. The co-terminality of the catheter 72 and stylet 130 enables the magnetic assembly 211 to function with the TLS sensor 50 in a TLS mode to track the position of the catheter distal tip 76A as it advances within the patient vasculature. Note, however, that for the tip confirmation functionality of the system 10, the distal end 230B of the stylet 130 need not be co-terminal with the catheter distal end 76A. Rather, all that is required is that a conductive path between the vasculature and the ECG sensor assembly 210 be established such that electrical impulses of the SA node or other node of the patient's heart can be detected. This conductive path in one embodiment can include various components including saline solution, blood, etc.

In one embodiment, once the catheter 72 has been introduced into the patient vasculature via the insertion site 73 (FIG. 1) the TLS mode of the system 10 can be employed to advance the catheter distal tip 76A toward its intended destination proximate the SA node. Upon approaching the region of the heart, ECG signals may be transmitted to the system 10 via the ECG sensor assembly 210. As the catheter 72 and the stylet 130 are advanced toward the patient's heart, the electrically conductive ECG sensor assembly 210, including the distal tip member 250, begins to detect the electrical impulses produced by the SA node. As such, the ECG sensor assembly 210 serves as an electrode for detecting the ECG signals.

The ECG sensor assembly 210 conveys the ECG signals to the TLS sensor 50. The ECG sensor assembly 210 is operably connected to the TLS sensor 50 via the tether connector 232. As described, the ECG signal can then be processed and depicted on the system display 30 (FIG. 1). Monitoring of the ECG signal received by the TLS sensor 50 and displayed by the display 30 enables a clinician to observe and analyze changes in the signal as the catheter distal tip 76A advances toward the SA node.

The ECG sensor assembly 210 and magnetic assembly 211 can work in concert in assisting a clinician in placing a catheter 72 within the vasculature. Generally, the magnetic assembly 211 of the stylet 130 assists the clinician in generally navigating the vasculature from initial catheter insertion so as to place the distal end 76A of the catheter 72 in the general region of the patient's heart. The ECG sensor assembly 210 can then be employed to guide the catheter distal end 76A to the desired location within the SVC by enabling the clinician to observe changes in the ECG signals produced by the heart as the ECG sensor assembly 210 approaches the SA node. Again, once a suitable ECG signal profile is observed, the clinician can determine that the distal ends of both the stylet 130 and the catheter 72 have arrived at the desired location with respect to the patient's heart. Once it has been positioned as desired, the catheter 72 may be secured in place and the stylet 130 removed from the catheter lumen.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A stylet (130) for placing a catheter (72) in a vasculature of a patient, comprising:
an ECG sensor assembly (210) including an electrode extending from a proximal end (230A) to a distal end (230B) of the stylet (130), the proximal end (230A) configured to couple with an ECG sensor (50);
a magnetic assembly (211) disposed along a distal tip portion (306) of the stylet (130), the magnetic assembly (211) producing a magnetic field;
a core wire (238) extending proximally away from the magnetic assembly (211); and
a coil (320) defining a lumen (327), the coil (320) extending along a distal portion (302) of the stylet (130),
wherein a distal portion of the core wire (238) is disposed within the lumen (327),
wherein the magnetic assembly (211) comprises a plurality of magnet elements (344) disposed within the lumen (327),
wherein one or more magnet elements (344) are attached to the coil (320).

2. The stylet (130) of claim 1, wherein the stylet (130) is configured to be inserted within a lumen of the catheter (72), and/or wherein the stylet (130) is configured for placement of the catheter (72) within a superior vena cava of the patient.

3. The stylet (130) of any of claims 1-2, wherein the electrode includes the core wire (238), and/or the coil (320).

4. The stylet (130) of any of claims 1-3, wherein each of the magnet elements (344) comprises a cylindrical shape, and wherein the magnet elements (344) are arranged end to end within the lumen.

5. The stylet (130) of any of claims 1-4, wherein the coil (320) is attached to the core wire (238), and/or electrically coupled with the core wire (238).

6. The stylet (130) of any of claims 1-5, wherein the coil (320) comprises a coil member (322) forming a first helix, preferably wherein the coil member (322) comprises a rectangular cross-sectional shape having a width and a thickness.

7. The stylet (130) of claim 6, wherein the coil member (322) is a first coil member, and wherein the coil (320) comprises at least a second coil member forming at least a second helix, preferably wherein the first coil member and the second coil member cross each other.

8. The stylet (130) of any of claims 1-7, wherein the coil (320) comprises at least three coil members (322) defining a braided or woven structure, and/or wherein the stylet (130) further comprises a sheath (330) extending along the distal portion, the sheath (330) covering the coil (320).

9. The stylet (130) of any of claims 1-8, wherein the core wire (238) includes a first thickness extending along a proximal portion of the core wire (238) and a second thickness extending along a distal portion of the core wire (238), wherein the second thickness is less than the first thickness, preferably wherein the core wire (238) includes a taper (313) extending between the first thickness the second thickness.

10. The stylet (130) of claim 9, wherein the distal portion of the core wire (238) is round, and/or extends along the magnet assembly.

11. The stylet (130) of any of claims 1-10, further comprising a distal tip member (350) coupled with the coil (320), preferably wherein the distal tip member (350) is formed of an electrically conductive material, more preferably wherein the distal tip member (350) is electrically coupled with the coil (320).

12. The stylet (130) of any of claims 1-11, wherein the core wire (238) extends distally beyond the magnetic assembly (211), preferably wherein the distal tip member (350) is electrically coupled with the core wire (238).

13. The stylet (130) of any of claims 1-12, further comprising a handle (236) attached to the core wire (238) at a proximal end of the core wire (238), and/or a tether (134) coupled with the core wire (238) at the proximal end of the core wire (238), wherein the tether (134) comprises an electrical conductor forming a portion of the electrode.

14. The stylet (130) of any of claims 6-13, wherein the width is between 0.01 mm and 1 mm, and/or wherein the thickness is between 0.01 mm and 1 mm.

15. The stylet (130) of any of claims 6-14, wherein a pitch of the first helix is between 0.01 mm and 10 mm.

16. An intravascular catheter assembly, comprising:
a catheter (72) comprising a lumen; and
the stylet (130) according to claim 1 disposed within the lumen of the catheter (72).

17. The catheter assembly of claim 16, wherein the catheter (72) is configured for placement of a tip of the catheter (72) within superior vena cava of a patient, and/or wherein a distal end of the catheter (72) and a distal end of the stylet (130) are substantially co-terminal.

18. The catheter assembly of any of claims 16-17, further comprising a preformed curve (410) along a distal portion of the catheter assembly, preferably wherein the preformed curve (410) is defined by a preform curve of the stylet (130).

19. The catheter assembly of any of claims 16-18, wherein the coil (320) of the stylet (130) is entirely disposed within the lumen, and/or wherein a sheath (330) of the stylet (130) is entirely disposed within the lumen.

## Patentansprüche

1. Mandrin (130) zum Platzieren eines Katheters (72) in einem Gefäßsystem eines Patienten, umfassend:
eine EKG-Sensoranordnung (210) einschließlich einer Elektrode, die sich von einem proximalen Ende (230A) zu einem distalen Ende (230B) des Mandrins (130) erstreckt, wobei das proximale Ende (230A) konfiguriert ist, um mit einem EKG-Sensor (50) gekoppelt zu werden;
eine magnetische Anordnung (211), die entlang eines distalen Spitzenabschnitts (306) des Mandrins (130) angeordnet ist, wobei die magnetische Anordnung (211) ein Magnetfeld erzeugt;
einen Kerndraht (238), der sich proximal von der magnetischen Anordnung (211) weg erstreckt; und
eine Spirale (320), die ein Lumen (327) definiert, wobei sich die Spirale (320) entlang eines distalen Abschnitts (302) des Mandrins (130) erstreckt,
wobei ein distaler Abschnitt des Kerndrahts (238) innerhalb des Lumens (327) angeordnet ist,
wobei die magnetische Anordnung (211) eine Vielzahl von Magnetelementen (344) umfasst, die innerhalb des Lumens (327) angeordnet sind,
wobei ein oder mehrere Magnetelemente (344) an der Spirale (320) angebracht sind.

2. Mandrin (130) nach Anspruch 1, wobei der Mandrin (130) konfiguriert ist, um in ein Lumen des Katheters (72) eingeführt zu werden, und/oder wobei der Mandrin (130) für die Platzierung des Katheters (72) in einer oberen Hohlvene des Patienten konfiguriert ist.

3. Mandrin (130) nach einem der Ansprüche 1-2, wobei die Elektrode den Kerndraht (238) und/oder die Spirale (320) einschließt.

4. Mandrin (130) nach einem der Ansprüche 1-3, wobei jedes der Magnetelemente (344) eine zylindrische Form umfasst, und wobei die Magnetelemente (344) innerhalb des Lumens Ende an Ende angeordnet sind.

5. Mandrin (130) nach einem der Ansprüche 1-4, wobei die Spirale (320) an dem Kerndraht (238) angebracht und/oder elektrisch mit dem Kerndraht (238) gekoppelt ist.

6. Mandrin (130) nach einem der Ansprüche 1-5, wobei die Spirale (320) ein Spiralenelement (322) umfasst, das eine erste Helix bildet, vorzugsweise wobei das Spiralenelement (322) eine rechteckige Querschnittsform umfasst, die eine Breite und eine Dicke aufweist.

7. Mandrin (130) nach Anspruch 6, wobei das Spiralenelement (322) ein erstes Spiralenelement ist, und wobei die Spirale (320) mindestens ein zweites Spiralenelement umfasst, das mindestens eine zweite Helix bildet, vorzugsweise wobei das erste Spiralenelement und das zweite Spiralenelement einander kreuzen.

8. Mandrin (130) nach einem der Ansprüche 1-7, wobei die Spirale (320) mindestens drei Spiralenelemente (322) umfasst, die eine geflochtene oder gewebte Struktur definieren, und/oder wobei der Mandrin (130) weiter eine Hülle (330) umfasst, die sich entlang des distalen Abschnitts erstreckt, wobei die Hülle (330) die Spirale (320) bedeckt.

9. Mandrin (130) nach einem der Ansprüche 1-8, wobei der Kerndraht (238) eine erste Dicke, die sich entlang eines proximalen Abschnitts des Kerndrahts (238) erstreckt, und eine zweite Dicke, die sich entlang eines distalen Abschnitts des Kerndrahts (238) erstreckt, einschließt, wobei die zweite Dicke geringer ist als die erste Dicke, vorzugsweise wobei der Kerndraht (238) eine Verjüngung (313) einschließt, die sich zwischen der ersten Dicke und der zweiten Dicke erstreckt.

10. Mandrin (130) nach Anspruch 9, wobei der distale Abschnitt des Kerndrahts (238) rund ist und/oder sich entlang der magnetischen Anordnung erstreckt.

11. Mandrin (130) nach einem der Ansprüche 1-10, weiter umfassend ein distales Spitzenelement (350), das mit der Spirale (320) gekoppelt ist, vorzugsweise wobei das distale Spitzenelement (350) aus einem elektrisch leitfähigen Material besteht, bevorzugter wobei das distale Spitzenelement (350) elektrisch mit der Spirale (320) gekoppelt ist.

12. Mandrin (130) nach einem der Ansprüche 1-11, wobei der Kerndraht (238) sich distal über die magnetische Anordnung (211) hinaus erstreckt, vorzugsweise wobei das distale Spitzenelement (350) elektrisch mit dem Kerndraht (238) gekoppelt ist.

13. Mandrin (130) nach einem der Ansprüche 1-12, weiter umfassend einen Griff (236), der an einem proximalen Ende des Kerndrahts (238) an dem Kerndraht (238) angebracht ist, und/oder ein Halteband (134), das mit dem Kerndraht (238) an dem proximalen Ende des Kerndrahts (238) gekoppelt ist, wobei das Halteband (134) einen elektrischen Leiter umfasst, der einen Abschnitt der Elektrode bildet.

14. Mandrin (130) nach einem der Ansprüche 6-13, wobei die Breite zwischen 0,01 mm und 1 mm beträgt, und/oder wobei die Dicke zwischen 0,01 mm und 1 mm beträgt.

15. Mandrin (130) nach einem der Ansprüche 6-14, wobei die Steigung der ersten Helix zwischen 0,01 mm und 10 mm beträgt.

16. Intravaskuläre Katheteranordnung, umfassend:
einen Katheter (72) umfassend ein Lumen; und
der Mandrin (130) nach Anspruch 1, angeordnet innerhalb des Lumens des Katheters (72).

17. Katheteranordnung nach Anspruch 16, wobei der Katheter (72) für die Platzierung einer Spitze des Katheters (72) in der oberen Hohlvene eines Patienten konfiguriert ist, und/oder wobei ein distales Ende des Katheters (72) und ein distales Ende des Mandrins (130) im Wesentlichen gleich enden.

18. Katheteranordnung nach einem der Ansprüche 16-17, weiter umfassend eine vorgeformte Kurve (410) entlang eines distalen Abschnitts der Katheteranordnung, vorzugsweise wobei die vorgeformte Kurve (410) durch eine Vorformkurve des Mandrins (130) definiert ist.

19. Katheteranordnung nach einem der Ansprüche 16-18, wobei die Spirale (320) des Mandrins (130) vollständig innerhalb des Lumens angeordnet ist, und/oder wobei eine Hülle (330) des Mandrins (130) vollständig innerhalb des Lumens angeordnet ist.

## Revendications

1. Stylet (130) permettant de placer un cathéter (72) dans un système vasculaire d'un patient, comprenant :
un ensemble capteur ECG (210) incluant une électrode s'étendant d'une extrémité proximale (230A) à une extrémité distale (230B) du stylet (130), l'extrémité proximale (230A) étant configurée pour se coupler avec un capteur ECG (50) ;
un ensemble magnétique (211) disposé le long d'une partie de pointe distale (306) du stylet (130), l'ensemble magnétique (211) produisant un champ magnétique ;
un fil d'âme (238) s'étendant de manière proximale à l'écart de l'ensemble magnétique (211) ; et
une bobine (320) définissant une lumière (327), la bobine (320) s'étendant le long d'une partie distale (302) du stylet (130),
dans lequel une partie distale du fil d'âme (238) est disposée à l'intérieur de la lumière (327),
dans lequel l'ensemble magnétique (211) comprend une pluralité d'éléments aimantés (344) disposés à l'intérieur de la lumière (327),
dans lequel un ou plusieurs éléments aimantés (344) sont fixés à la bobine (320).

2. Stylet (130) selon la revendication 1, dans lequel le stylet (130) est configuré pour être inséré à l'intérieur d'une lumière du cathéter (72), et/ou dans lequel le stylet (130) est configuré pour un placement du cathéter (72) à l'intérieur d'une veine cave supérieure du patient.

3. Stylet (130) selon l'une quelconque des revendications 1 et 2, dans lequel l'électrode inclut le fil d'âme (238), et/ou la bobine (320).

4. Stylet (130) selon l'une quelconque des revendications 1 à 3, dans lequel chacun des éléments aimantés (344) comprend une forme cylindrique, et dans lequel les éléments aimantés (344) sont agencés bout à bout à l'intérieur de la lumière.

5. Stylet (130) selon l'une quelconque des revendications 1 à 4, dans lequel la bobine (320) est fixée au fil d'âme (238), et/ou électriquement couplée avec le fil d'âme (238).

6. Stylet (130) selon l'une quelconque des revendications 1 à 5, dans lequel la bobine (320) comprend un élément de bobine (322) formant une première hélice, de préférence dans lequel l'élément de bobine (322) comprend une forme de section transversale rectangulaire présentant une largeur et une épaisseur.

7. Stylet (130) selon la revendication 6, dans lequel l'élément de bobine (322) est un premier élément de bobine, et dans lequel la bobine (320) comprend au moins un second élément de bobine formant au moins une seconde hélice, de préférence dans lequel le premier élément de bobine et le second élément de bobine se croisent.

8. Stylet (130) selon l'une quelconque des revendications 1 à 7, dans lequel la bobine (320) comprend au moins trois éléments de bobine (322) définissant une structure tressée ou tissée, et/ou dans lequel le stylet (130) comprend en outre une gaine (330) s'étendant le long de la partie distale, la gaine (330) recouvrant la bobine (320).

9. Stylet (130) selon l'une quelconque des revendications 1 à 8, dans lequel le fil d'âme (238) inclut une première épaisseur s'étendant le long d'une partie proximale du fil d'âme (238) et une seconde épaisseur s'étendant le long d'une partie distale du fil d'âme (238), dans lequel la seconde épaisseur est inférieure à la première épaisseur, de préférence dans lequel le fil d'âme (238) inclut un effilement (313) s'étendant entre la première épaisseur et la seconde épaisseur.

10. Stylet (130) selon la revendication 9, dans lequel la partie distale du fil d'âme (238) est ronde, et/ou s'étend le long de l'ensemble aimanté.

11. Stylet (130) selon l'une quelconque des revendications 1 à 10, comprenant en outre un élément de pointe distale (350) couplé avec la bobine (320), de préférence dans lequel l'élément de pointe distale (350) est formé d'un matériau électriquement conducteur, plus préférentiellement dans lequel l'élément de pointe distale (350) est électriquement couplé avec la bobine (320).

12. Stylet (130) selon l'une quelconque des revendications 1 à 11, dans lequel le fil d'âme (238) s'étend de manière distale au-delà de l'ensemble magnétique (211), de préférence dans lequel l'élément de pointe distale (350) est électriquement couplé avec le fil d'âme (238).

13. Stylet (130) selon l'une quelconque des revendications 1 à 12, comprenant en outre une poignée (236) fixée au fil d'âme (238) au niveau d'une extrémité proximale du fil d'âme (238), et/ou une attache (134) couplée avec le fil d'âme (238) au niveau de l'extrémité proximale du fil d'âme (238), dans lequel l'attache (134) comprend un conducteur électrique formant une partie de l'électrode.

14. Stylet (130) selon l'une quelconque des revendications 6 à 13, dans lequel la largeur est comprise entre 0,01 mm et 1 mm, et/ou dans lequel l'épaisseur est comprise entre 0,01 mm et 1 mm.

15. Stylet (130) selon l'une quelconque des revendications 6 à 14, dans lequel un pas de la première hélice est compris entre 0,01 mm et 10 mm.

16. Ensemble cathéter intravasculaire, comprenant :
un cathéter (72) comprenant une lumière ; et
le stylet (130) selon la revendication 1 disposé à l'intérieur de la lumière du cathéter (72).

17. Ensemble cathéter selon la revendication 16, dans lequel le cathéter (72) est configuré pour un placement d'une pointe du cathéter (72) à l'intérieur d'une veine cave supérieure d'un patient, et/ou dans lequel une extrémité distale du cathéter (72) et une extrémité distale du stylet (130) sont sensiblement co-terminales.

18. Ensemble cathéter selon l'une quelconque des revendications 16 et 17, comprenant en outre une courbure préformée (410) le long d'une partie distale de l'ensemble cathéter, de préférence dans lequel la courbure préformée (410) est définie par une courbe de préforme du stylet (130).

19. Ensemble cathéter selon l'une quelconque des revendications 16 à 18, dans lequel la bobine (320) du stylet (130) est entièrement disposée à l'intérieur de la lumière, et/ou dans lequel une gaine (330) du stylet (130) est entièrement disposée à l'intérieur de la lumière.
